# EUROPEAN PATENT SPECIFICATION

(11) **EP 2 742 140 B1**
(45) Date of publication and mention of the grant of the patent: **16.11.2016**
(21) Application number: 12766726.9
(22) Date of filing: 07.08.2012
(51) Int. Cl.: C12N 15/70, C12N 15/73

(54) **PROKARYOTIC HOST CELL COMPRISING EXPRESSION VECTOR**
PROKARYOTISCHE WIRTSZELLE MIT EXPRESSIONSVEKTOR
CELLULE HÔTE PROCARYOTE COMPRENANT UN VECTEUR D'EXPRESSION

(30) Priority: 11.08.2011 PL 39593711
(43) Date of publication of application: 18.06.2014
(73) Proprietor: Instytut Biotechnologii I Antybiotykow, 02-516 Warszawa (PL)
(72) Inventor: KESIK-BRODACKA, Malgorzata, 00-137 Warszawa (PL); ROMANIK, Agnieszka, 07-300 Ostrów Mazowiecki (PL); PLUCIENNICZAK, Andrzej, 04-360 Warszawa (PL); PLUCIENNICZAK, Grazyna, 04-360 Warszawa (PL); MIKIEWICZ-SYGULA, Diana, 03-337 Warszawa (PL)
(74) Representative: Witek, Rafal
(86) International application number: PCT/PL2012/000064
(87) International publication number: WO 2013/022358

(56) References cited:
- WO-A1-2005/052151
- WO-A2-2007/142547
- ROGERS ET AL: "Amplification of the aroA gene from Escherichia coli results in tolerance to the herbicide glyphosate.", APPLIED AND ENVIRONMENTAL MICROBIOLOGY, vol. 46, no. 1, 1 July 1983 (1983-07-01), pages 37-43, XP055045944, ISSN: 0099-2240
- RL THUNE ET AL: "Construction of a safe, stable, efficacious vaccine against Photobacterium damselae ssp. piscicida", DISEASES OF AQUATIC ORGANISMS, vol. 57, no. 1-2, 1 December 2003 (2003-12-01), pages 51-58, XP055045954, ISSN: 0177-5103, DOI: 10.3354/dao057051
- M. SCHMITZ ET AL: "Pulse Experiments as a Prerequisite for the Quantification of in Vivo Enzyme Kinetics in Aromatic Amino Acid Pathway of Escherichia coli", BIOTECHNOLOGY PROGRESS, vol. 18, no. 5, 4 October 2002 (2002-10-04), pages 935-941, XP055045957, ISSN: 8756-7938, DOI: 10.1021/bp010199z
- FIEDLER M ET AL: "proBA complementation of an auxotrophic E. coli strain improves plasmid stability and expression yield during fermenter production of a recombinant antibody fragment", GENE, ELSEVIER, AMSTERDAM, NL, vol. 274, no. 1-2, 22 August 2001 (2001-08-22), pages 111-118, XP004319600, ISSN: 0378-1119, DOI: 10.1016/S0378-1119(01)00629-1
- VIDAL ET AL: "Development of an antibiotic-free plasmid selection system based on glycine auxotrophy for recombinant protein overproduction in Escherichia coli", JOURNAL OF BIOTECHNOLOGY, ELSEVIER SCIENCE PUBLISHERS, AMSTERDAM, NL, vol. 134, no. 1-2, 24 January 2008 (2008-01-24), pages 127-136, XP022500135, ISSN: 0168-1656, DOI: 10.1016/J.JBIOTEC.2008.01.011

### SEQUENCE LISTING

<110> Instytut Biotechnologii i Antybiotykow (institute Biotechnology and Antibiotics)
<120> Expression cassette, use of the expression cassette, vector, host cell, bacterial strain and a method for producing a polypeptide
<130> 22459/PCT/12
<140> PCT/PL2012/0000
   <141> 2012-08-06
<150> PL395937
   <151> 2011-08-11
<160> 21
<170> PatentIn version 3.5
<210> 1
   <211> 44
   <212> DNA
   <213> Artificial Sequence
<220>
   <223> primer AroA1
<400> 1
   gctggcggca ttttggccgc tagagagttg agttcatgga atcc 44
<210> 2
   <211> 63
   <212> DNA
   <213> Artificial Sequence
<220>
   <223> primer AroA2
<400> 2
   ggggtcgacc taagcggccg ctaatcccac agccgccagt tccgctggcg gcattttggc 60
   cgc 63
<210> 3
   <211> 37
   <212> DNA
   <213> Artificial Sequence
<220>
   <223> primer AroAk
<400> 3
   aaaaagctta ggctgcctgg ctaatccgcg ccagctg 37
<210> 4
   <211> 27
   <212> DNA
   <213> Artificial Sequence
<220>
   <223> primer AroNdeD
<400> 4
   gcattcagca tgtggcgcac gtcatcg 27
<210> 5
   <211> 32
   <212> DNA
   <213> Artificial Sequence
<220>
   <223> primer ZaAROA
<400> 5
   cgctaccatt aatcaaaatg actcacaagg tc 32
<210> 6
   <211> 24
   <212> DNA
   <213> Artificial Sequence
<220>
   <223> primer PrzeAROA
<400> 6
   ggttgagttc gaacgccgtc acgg 24
<210> 7
   <211> 22
   <212> DNA
   <213> Artificial Sequence
<220>
   <223> primer Aro700R
<400> 7
   cgacaaattg ttgatagtgc tg 22
<210> 8
   <211> 52
   <212> DNA
   <213> Artificial Sequence
<220>
   <223> primer AroA1-SDkm
<400> 8
   gctggcggca ttttggccgc aaggggtgtt atggaatccc tgacgttaca ac 52
<210> 9
   <211> 58
   <212> DNA
   <213> Artificial Sequence
<220>
   <223> primer AroA1-SD-AGGAGG
<400> 9
   gctggcggca ttttggccgc ataaaggagg taaataatgg aatccctgac gttacaac 58
<210> 10
   <211> 45
   <212> DNA
   <213> Artificial Sequence
<220>
   <223> primer AroA1zG
<400> 10
   gctggcggca ttttggccgc gtagagagtt gagttcatgg aatcc 45
<210> 11
   <211> 30
   <212> DNA
   <213> Artificial sequence
<220>
   <223> primer PA-4DP
<400> 11
   ggggaattca tatgaaacgt tttcattatg 30
<210> 12
   <211> 82
   <212> DNA
   <213> Artificial sequence
<220>
   <223> primer PAzAroA
<400> 12
   ccaaaatgcc gccagcggaa ctggcggctg tgggattagc ggccgcttag gtcgacttat 60
   cctatctcat agcctttttt ag 82
<210> 13
   <211> 40
   <212> DNA
   <213> Artificial Sequence
<220>
   <223> primer PA4DKXho
<400> 13
   aaaagcttct cgagttatcc tatctcatag ccttttttag 40
<210> 14
   <211> 32
   <212> DNA
   <213> Artificial Sequence
<220>
   <223> primer PA4DK
<400> 14
   aaaagcttat cctatctcat agcctttttt ag 32
<210> 15
   <211> 1800
   <212> DNA
   <213> Artificial Sequence
<220>
   <223> sequence of expression cassette included in vector pIGKesAroAPA-G
<400> 15
   catatgaaac gttttcatta tgatcgcaat aacataccca gttggggcgg atgagtcagt 60
   agttaaggag gctcatagag aagtaattaa ttcgtcaaca gagggattat tgttaaatat 120
   tgataaggat ataagaaaaa tattatcagg ttatattgta gaaattgaag atactgaagg 180
   gcttaaagaa gttataaatg acagatatga tatgttgaat atttctagtt tacggcaaga 240
   tggaaaaaca tttatagatt ttaaaaaata taatgataaa ttaccgttat atataagtaa 300
   tcccaattat aaggtaaatg tatatgctgt ttctatagaa aacactatta ttaatcctag 360
   tgagaatggg gatactagta ccaacgggat caagaaaatt ttaatctttt ctaaaaaagg 420
   ctatgagata ggataagtcg acctaagcgg ccgctaatcc cacagccgcc agttccgctg 480
   gcggcatttt ggccgcgtag agagttgagt tcatggaatc cctgacgtta caacccatcg 540
   ctcgtgtcga tggcactatt aatctgcccg gttccaagag cgtttctaac cgcgctttat 600
   tgctggcggc attagcacac ggcaaaacag tattaaccaa tctgctggat agcgatgacg 660
   tgcgccatat gctgaatgca ttaacagggt taggggtaag ctatacgctt tcagccgatc 720
   gtacgcgttg cgaaattatc ggtaacggcg gtccattaca cgcagaaggt gccctggagt 780
   tgttcctcgg taacgccgga acggcaatgc gtccgctggc ggcagctctt tgtctgggta 840
   gcaatgatat tgtgctgacc ggtgagccgc gtatgaaaga acgcccgatt ggtcatctgg 900
   tggatgctct gcgcctgggc ggggcgaaga tcacttacct ggaacaagaa aattatccgc 960
   cgttgcgttt acagggcggc tttaccggcg gcaacgttga cgttgatggc tccgtttcca 1020
   gccaattcct caccgcactg ttaatgactg cgcctcttgc gccggaagat acggtgattc 1080
   gtattaaagg cgatctggtt tctaaacctt atatcgacat cacactcaat ctgatgaaga 1140
   cgtttggtgt tgaaattgaa aatcagcact atcaacaatt tgtcgtaaaa ggcgggcagt 1200
   cttatcagtc tccgggtact tatttggtcg aaggcgatgc atcttcggct tcttactttc 1260
   tggcagcagc agcaatcaaa ggcggcactg taaaagtgac cggtattgga cgtaacagta 1320
   tgcagggtga tattcgcttt gctgatgtgc tggaaaaaat gggcgcgacc atttgctggg 1380
   gcgatgatta tatttcctgc acgcgtggtg aactgaacgc tattgatatg gatatgaacc 1440
   atattcccga tgcggcgatg accattgcca cggcggcgtt atttgcaaaa ggcaccacca 1500
   cgctgcgcaa tatctataac tggcgtgtta aagaaaccga tcgcctgttt gcgatggcaa 1560
   cagaactgcg taaagtcggt gcggaagtag aagaggggca cgattacatt cgtatcactc 1620
   caccggaaaa actgaacttt gccgagatcg cgacatacaa tgatcaccgg atggcgatgt 1680
   gtttctcgct ggtggcgttg tcagatacac cagtgacgat tcttgatccc aaatgcacgg 1740
   ccaaaacatt tccggattat ttcgagcagc tggcgcggat tagccaggca gcctaagctt 1800
<210> 16
   <211> 1800
   <212> DNA
   <213> Artificial Sequence
<220>
   <223> sequence of expression cassette included in vector pIGKesAroAPA-AGG
<400> 16
   catatgaaac gttttcatta tgatcgcaat aacataccca gttggggcgg atgagtcagt      60
   agttaaggag gctcatagag aagtaattaa ttcgtcaaca gagggattat tgttaaatat     120
   tgataaggat ataagaaaaa tattatcagg ttatattgta gaaattgaag atactgaagg     180
   gcttaaagaa gttataaatg acagatatga tatgttgaat atttctagtt tacggcaaga     240
   tggaaaaaca tttatagatt ttaaaaaata taatgataaa ttaccgttat atataagtaa     300
   tcccaattat aaggtaaatg tatatgctgt ttctatagaa aacactatta ttaatcctag     360
   tgagaatggg gatactagta ccaacgggat caagaaaatt ttaatctttt ctaaaaaagg     420
   ctatgagata ggataagtcg acctaagcgg ccgctaatcc cacagccgcc agttccgctg     480
   gcggcatttt ggccgcataa aggaggtaaa taatggaatc cctgacgtta caacccatcg     540
   ctcgtgtcga tggcactatt aatctgcccg gttccaagag cgtttctaac cgcgctttat     600
   tgctggcggc attagcacac ggcaaaacag tattaaccaa tctgctggat agcgatgacg     660
   tgcgccacat gctgaatgca ttaacagggt taggggtaag ctatacgctt tcagccgatc     720
   gtacgcgttg cgaaattatc ggtaacggcg gtccattaca cgcagaaggt gccctggagt     780
   tgttcctcgg taacgccgga acggcaatgc gtccgctggc ggcagctctt tgtctgggta     840
   gcaatgatat tgtgctgacc ggtgagccgc gtatgaaaga acgcccgatt ggtcatctgg     900
   tggatgctct gcgcctgggc ggggcgaaga tcacttacct ggaacaagaa aattatccgc     960
   cgttgcgttt acagggcggc tttaccggcg gcaacgttga cgttgatggc tccgtttcca    1020
   gccaattcct caccgcactg ttaatgactg cgcctcttgc gccggaagat acggtgattc    1080
   gtattaaagg cgatctggtt tctaaacctt atatcgacat cacactcaat ctgatgaaga    1140
   cgtttggtgt tgaaattgaa aatcagcact atcaacaatt tgtcgtaaaa ggcgggcagt    1200
   cttatcagtc tccgggtact tatttggtcg aaggcgatgc atcttcggct tcttactttc    1260
   tggcagcagc agcaatcaaa ggcggcactg taaaagtgac cggtattgga cgtaacagta    1320
   tgcagggtga tattcgcttt gctgatgtgc tggaaaaaat gggcgcgacc atttgctggg    1380
   gcgatgatta tatttcctgc acgcgtggtg aactgaacgc tattgatatg gatatgaacc    1440
   atattcccga tgcggcgatg accattgcca cggcggcgtt atttgcaaaa ggcaccacca    1500
   cgctgcgcaa tatctataac tggcgtgtta aagaaaccga tcgcctgttt gcgatggcaa    1560
   cagaactgcg taaagtcggt gcggaagtag aagaggggca cgattacatt cgtatcactc    1620
   caccggaaaa actgaacttt gccgagatcg cgacatacaa tgatcaccgg atggcgatgt    1680
   gtttctcgct ggtggcgttg tcagatacac cagtgacgat tcttgatccc aaatgcacgg    1740
   ccaaaacatt tccggattat ttcgagcagc tggcgcggat tagccaggca gcctaagctt    1800
<210> 17
   <211> 21
   <212> DNA
   <213> Artificial Sequence
<220>
   <223> SD
<400> 17
   ggccgctaga gagttgagtt c 21
<210> 18
   <211> 22
   <212> DNA
   <213> Artificial Sequence
<220>
   <223> SD-G
<400> 18
   ggccgcgtag agagttgagt tc 22
<210> 19
   <211> 22
   <212> DNA
   <213> Artificial Sequence
<220>
   <223> SD-AGG
<400> 19
   ggccgcataa aggaggtaaa ta 22
<210> 20
   <211> 16
   <212> DNA
   <213> Artificial Sequence
<220>
   <223> SD-Km
<400> 20
   ggccgcaagg ggtgtt 16
<210> 21
   <211> 871
   <212> DNA
   <213> Artificial Sequence
<220>
   <223> sequence of the cloned insert PA-4D in vector pIGT7PA
<400> 21
   catatgaaac gttttcatta tgatagaaat aacatagcag ttggggcgga tgagtcagta       60
   gttaaggagg ctcatagaga agtaattaat tcgtcaacag agggattatt gttaaatatt      120
   gataaggata taagaaaaat attatcaggt tatattgtag aaattgaaga tactgaaggg      180
   cttaaagaag ttataaatga cagatatgat atgttgaata tttctagttt acggcaagat      240
   ggaaaaacat ttatagattt taaaaaatat aatgataaat taccgttata tataagtaat      300
   cccaattata aggtaaatgt atatgctgtt actaaagaaa acactattat taatcctagt      360
   gagaatgggg atactagtac caacgggatc aagaaaattt taatcttttc taaaaaaggc      420
   tatgagatag gataaatgaa acgttttcat tatgatagaa ataacatagc agttggggcg      480
   gatgagtcag tagttaagga ggctcataga gaagtaatta attcgtcaac agagggatta      540
   ttgttaaata ttgataagga tataagaaaa atattatcag gttatattgt agaaattgaa      600
   gatactgaag ggcttaaaga agttataaat gacagatatg atatgttgaa tatttctagt      660
   ttacggcaag atggaaaaac atttatagat tttaaaaaat ataatgataa attaccgtta      720
   tatataagta atcccaatta taaggtaaat gtatatgctg ttactaaaga aaacactatt      780
   attaatccta gtgagaatgg ggatactagt accaacggga tcaagaaaat tttaatcttt      840
   tctaaaaaag gctatgagat aggataagct t                                     871

## Description

The subject matters of invention relate to expression cassette, use of the expression cassette, expression vector, prokaryotic host cell comprising expression vector, bacterial strain and a method for producing a polypeptide. In particular the invention concerns stable expression of recombinant proteins without the need of antibiotic use, and especially expression of recombinant proteins obtained with the use of especially effective T7 phage polymerase/promoter system. Only small percentage of living organism proteins is present in their cells in quantities sufficient to enable direct isolation on a large scale. The possibility to produce in prokaryotic systems peptides, which were difficult and/or expensive to derive from natural sources, was a major breakthrough in biotechnology. This procedure was established after a discovery from the 1980's in which a system based on RNA polymerases depending on DNA was incorporated into host cells. Such cells are transformed with vectors with promoters recognised by these polymerases. One of the most widely used systems of this type is a system in which polymerase T7 recognises the promoter of T7 phage. In this system sequence encoding T7 polymerase is present in the bacterial chromosome and the recognised promoter sequence is present in the expression vector.

Such an expression system, vector and a method of expression target polypeptide were described for example in United States patents no. 4.952.496 and 5.693.489. In the solutions described in the cited patent descriptions, vector comprising target gene functionally linked to the promoter recognized by the RNA polymerase from T7 phage is introduced to host cell, such as *Escherichia coli,* containing gene coding phage T7 RNA polymerase. The host cells are cultivated in the known conditions enabling expression.

In many cases the system prove to be very effective and permit obtaining high level of expression, with the target protein comprising even 50% of the total cell protein production [Metlitskaia L, Cabralda JE, Suleman D, Kerry C, Brinkman J, Bartfeld D, Guarna MM, Recombinant antimicrobial peptides efficiently produced using novel cloning and purification processes, Biotechnol. Appl. Biochem., (2004 Jun); 39 (Pt 3):339-45]. However, problems are also encounter in this area. One of them is the gradual decrease in the target gene expression controlled by T7 phage promoter. This problem has not been solved since the beginning of use of systems comprising T7 phage promoters.

It was previously thought that decrease in gene expression is caused by loss or mutations of plasmid bearing the target gene. It was suggested to test the population of bacteria on their capacity to express high levels of the target protein [Studier FW, Moffatt BA, Use of bacteriophage T7 RNA polymerase to direct selective high-level expression of cloned genes, J. Mol. Biol. (1986) 189; 113-130; Studier FW, Rosenberg AH, Dunn JJ, Dubendorff JW, Use of T7 RNA polymerase to direct expression of cloned genes, Meth. Enzymol. (1990) 185 : 60-89].

Vethanayagam et al. for the first time found and described the major contributing factor to decreased expression levels in T7 based system [Vethanayagam JG and Flower AM, Decreased gene expression from T7 promoters may be due to impaired production of active T7 RNA polymerase, Microb Cell Fac.( 2005 Jan 7);4:3]. It is connected with chromosomal mutations occurring in the area of sequence coding DNA-dependent RNA polymerase of T7 phage.

Another problem of the widely used expression system based on the T7 phage polymerase/promoter system, as well as other expression systems, is widespread use of genes granting antibiotic resistance to bacteria. Those genes are placed within expression vectors and are used as selection markers both for selection during cloning and for cultivation of bacteria strains with introduced recombinant DNA. From this stems the necessity to use antibiotics during cultivation of these strains. The use of antibiotics markedly increases production costs of the target protein, both because of the cost of the antibiotic itself, and the because of the cost of utilisation of post-cultivation waste.

The patent application PL379905 (published 2007.12.10) describes expression cassette and vector for expression system based on a phage polymerase and promoter, where phage promoter regulates synthesis of the target polypeptide and produces a protein serving as a selection factor, whose production is necessary for survival of the prokaryotic host cell. This allows elimination of cells, in which there is no expression controlled by the phage promoter. This application also describes a cell of a prokaryotic host, which contains an expression vector and a method of obtaining of the polypeptide and the use of the expression cassette.

The decrease in time of the expression level of the recombinant proteins and the necessity of antibiotic use during the cultivation of bacteria make the conventional expression system using T7 phage polymerase and promoter not an advantageous choice during planning of industry-scale expression.

The invention solves the problems indicated above, especially the gradual decrease in the target gene expression and enables stable expression of the target polypeptide. The stable expression of the target polypeptide is obtained without the need of antibiotic use.

Unexpectedly, it turned out, that the construction of the expression cassette ensures expression of the target polypeptide in a prokaryotic host at a level not lower than in a standard system using only T7 phage promoter. It is possible because high expression, characteristic for phage promoters, especially T7 phage promoter, is directed mainly at the target polypeptide. On the other hand expression level of the selection marker is high enough to ensure host cell survival. This was achieved by presence of transcription terminator from promoters other than phage promoters upstream of 5' end of the gene encoding selection marker, and in the favourable variant by including upstream of the transcription terminator a translational stop codon and by modifying non-coding part of the selection marker-encoding gene at the 5' end (part containing Shine-Dalgarno sequence).
Additionally on the 5' end there is placed an originally developed during the conducted research, non-coding part of sequence of the gene encoding the selection factor (the part containing Shine-Dalgarno sequence) for aroA gene. The Shine-Dalgarno (SD) sequence provided in the available literature [Dunkan K., Coggins J. R. The serC-aroA operon of Eshcelichia coli A mixed function poeron encoding enzymem from two different aminoacid biosynthetic pathways. (1986) Biochem. J.] does not perform its role, when placed in the invention/described expression cassette. Additionally a selected non-coding part of sequence of the gene encoding the selection factor was modified to obtain a sequence variant providing optimal level of expression of aroA gene encoding EPSPS protein. There was also developed a variant of synthetic sequence of this type. It also enables expression of EPSPS protein at the appropriate level.
According to the invention problem of stable, high level expression in systems where DNA-dependent RNA polymerase and promoter recognised by this polymerase are used, was solved by developing of system wherein polymerase recognises promoter regulating synthesis of target protein as well as synthesis of selection marker, which is required for survival of the host.
Therefore, in the culture conducted with the use of the developed systems, there are selected only these cells, which bear the plasmid and at the same time have functional T7 phage polymerase/promoter system. The selection factor, whose production is depending on efficient functioning of T7 phage polymerase/promoter system is a chemical compound complementing genetic defect of the host cell.
It has been established that the invented expression cassette is functional if selection marker production is controlled solely by phage promoter recognised specifically by phage polymerase. Therefore upstream of the 5' end of the gene encoding the selection marker there is cloned-in transcription terminator from promoters other than phage promoters. This prevents expression of the gene encoding the selection marker by promoters present in the expression vector and recognised by host's polymerases.
Inclusion of the sequence encoding the selection marker such that its expression is dependent solely on phage's promoter causes stoppage of production of this protein, which is required for cell's survival and elimination of cells which, due to a mutation, have lost ability to produce a functional polymerase and/or those in which, due to a mutation, the phage promoter have lost its functionality.
WO2007142547 discloses an expression cassette comprising a phage promoter functionally linked to a sequence encoding a target polypeptide and a selection marker, which is required for the survival of the host expressing the target polypeptide, where the transcriptional termination sequence located between the target sequence and the selectable marker sequence is inconsistently recognized by the phage DNA-dependent RNA polymerase. The selection marker being required for the survival of the host expressing the target polypeptide is preferably selected from markers which introduce antibiotic resistance and markers which complement the genetic defect of the host cell or their functional fragments. However, WO2007142547 does not disclose or suggest which of the multiple combination are going to work in a specific recombinant host.
The subject of the invention is a prokaryotic host cell containing an expression cassette containing a phage promoter and a sequence encoding the target polypeptide, where the cassette has the formula:

P-X-S-T-M

where: P denotes the T7 phage promoter sequence, X denotes the target polypeptide sequence, S denotes the translation stop codon, T denotes transcription terminator for promoters other than the T7 phage promoters and M denotes the sequence encoding the selection factor necessary for survival of the host expressing the target polypeptide and complementing genetic defect of the host cell, the sequence encoding the selection factor is the *aroA* sequence encoding 5-enolpyruvylshikimate-3-phosphate synthase (EPSPS), and the host for the vector containing the cassette is E. coli strain BL21 (DE3) with functional deletion of the *aroA* gene.

Preferentially, in the host cell according to the invention, the transcription terminator for promoters other than the phage promoter is a tryptophan terminator.

In the conducted experiments, whose results formed the basis for developing the invention, there was used a plasmid vector containing a promoter originating from T7 phage.

Into this vector there was incorporated the described expression cassette. An expression vector was obtained, which enabled efficient and stable expression of the recombinant protein without the need for use of antibiotic pressure in *E. coli* BL21(DE3)AaroA cells. An expression vector used for insertion of the expression cassette can be any plasmid, which is maintained in given host cell. Such a vector must have an appropriate promoter. Such a promoter can be any promoter, both constitutive and induced, but it must be a promoter from the group of promoters read by polymerases not originating from the bacteria. At the 3' end of the promoter sequence according to the invention there is the described expression cassette enabling efficient and stable expression of the target protein. At the same time, this promoter will control expression of the factor complementing the genetic defect of the host.

A new *E. coli* bacteria strain performing the function of the host was being developed. This strain has characteristics necessary for correct functioning of the developed system. The host is appropriately modified BL21(DE3) *E. coli* strain. It is the strain BL21(DE3) ΔaroA.

In the chromosome of *E. coli* BL21(DE3) strain and its derivatives there is a gene encoding RNA polymerase of T7 phage under the control of *lacUV5* promoter. The gene is recognised by the bacterial RNA polymerase. There is an operator sequence between the promoter and the gene encoding T7 RNA polymerase, which binds repressor protein in that manner blocking the initiation of transcription. Only when repressor binds to IPTG, which leads to loss of protein affinity to operator site, that the transcription of phage polymerase gene may start.

In the case of using this system for large scale production, it would be advantageous to employ alternative method of induction, so called autoinduction [Studiem F. W. Protein production by auto-induction In high density shaking cultures. Protein Expo. Purif. 41, 207-234 (2005)]. This method is based on the bacteria's ability to use various sources of carbon and to negative regulation of gene expression by catabolic repression.

BL21(DE3) bacteria strain has been given characteristics enabling its use in the disclosed expression system. This has been achieved by introducing a functional deletion of one of the cell's chromosomal genes, which is an example of a gene belonging to the group of genes whose products are necessary for survival of the host cell when appropriate culture conditions are employed. This caused metabolic defect. Because of that defect one or more of metabolites important for the cell's life are not synthesised.

The chosen chromosomal gene, whose deletion has been conducted is aroA gene encoding 3-phosphoshikimate 1-carboxyvinyltransferase (other used name is 5-enolpyruvylshikimate-3-phosphate synthase, EPSPS). EPSPS protein is built of 428 amino acids; its molecular mass is 46200 Da. EPSPS protein participates in biosynthesis path of aromatic amino acids L-tryptophane, L-tyrosine, L- phenyloalanine. It is a gene, whose protein product is necessary for survival of the bacteria cell. Inactivation of AroA gene encoding EPSPS protein leads to unconditional auxotrophy [Monitoring of gene knockout: genome-wide of conditionally essential genes. Lisa K Smith, et al.]. Bacteria with functional deletion of aroA gene require for their growth presence in the media of aromatic amino acids: L-tryptophane, L-tyrosine and L-phenyloalanine and aromatic compounds: 4-hydroxybenzoic acid, 4-aminobenzoic acid, 2,3-dihydroxybenzoic acid. aroA gene is an example of a gene, whose deletion leads to obtaining of *E. coli* strain with new characteristics. In the literature there so far has been no information about obtaining of BL21(DE3) strain *E. coli* bacteria with functional deletion of aroA gene. There have also been no information indicating the use *of E. coli* bacteria, and in particular BL21(DE3) strain *E. coli* with deletion of this or any other gene resulting in obtaining auxotrophic mutants, for stable expression of recombinant proteins and selection of cells expressing recombinant proteins. After introduction via transformation of the expression vector with the expression cassette into cells of *E. coli* BL21(DE3) ΔaroA strain, there occurs expression of the target protein and EPSPS protein under the control of T7 phage promoter. Due to expression of EPSPS protein dependent on proper functioning of T7 phage polymerase/promoter system, there is possible the stable expression of target proteins due to selection of cells expressing the target proteins and the selection factor. This mechanism fulfils first of the assumptions of the invention.

In effect there was obtained a system comprising of the expression vector and properly prepared host cell (bacteria strain), which is properly prepared *E. coli* BL(21)DE3 strain. This system enables obtaining of a stable in time expression of the target protein. Stable in time expression of the target protein increases efficiency of production of this protein from a given volume of culture media, therefore notably decreasing its production cost.

Second advantage of the developed system is the lack of necessity of employing of genes granting resistance to antibiotics as selection markers necessary for retaining plasmids in the cell. This allows fulfilling safety requirements concerning non-proliferation in the environment of genes responsible for resistance to antibiotics. Additionally this allows avoiding the issue, that in some therapeutic uses of the purified protein employment of antibiotics can be undesirable. Lack of necessity of employing antibiotic pressure at any stage of the fermentation process will significantly lower costs of possible industry-scale production of the recombinant protein. Also eliminated is the problem of utilisation of antibiotic-containing waste. Ability to conduct the culture and obtain efficient expression of recombinant proteins according to the described invention fulfils the second assumption of the invention.

The proposed invention will enable use of extraordinarily efficient T7 phage polymerase/promoter system for production of recombinant proteins not only on laboratory scale, as it is employed currently, but also for industry scale production.

Choice of the *E. coli* chromosomal gene, whose functional deletion has been performed is not random, nor is it obvious. There were a number of attempts made in order to obtain optimal variant of a strain to be used in the system being the subject of the invention. There were a number of factors which made choice of the appropriate gene or group of genes difficult. First, despite the fact that complete genome sequence has been established for two distinct *E. coli* strains MG1665 i W3110 [Aiba H., Baba T., Hayashi H., Inada T., Isono K., Itoh T., Kasai H., Kashimoto K., Kiura S., Kitakawa M. (996) A 570-kb DNA sequence of the Eschelichia coli K-12 genome corresponding to the 28.0-40.1 min region on the linkage map. DNA Res., 3:363-377; Ithon T., Aiba H., Baba T., Hayashi K., Inada T., Isono K., Kasai H., Kiura S., Kitakawa M., (1996) A 460-kb DNA sequence of the Escherichia coli K-12 genome corresponding to the 40.1-50.0 min region on the linkage map. DNA Res., 3:379-392; Oshima T., Aiba H., Baba T., Fujita K., Hayashi K., Honjo A., Ikemoto K., Inada T., Itoh T., Kajihara M., (1996) A 718-kb DNA sequence of the Escherichia coli K-12 genome corresponding to the 12.7-28.0 min region on the linkage map. DNA Res., 3:137-155; Blattner F.R., Plunkett G., Bloch C.A., Perna N., Burland V, Rile M., Collado-Vides J., Glasner J. D., Rode C. K., Mayhew G. F. (1997) The complete genome sequence of Escherichia coli K-12. Science, 277:1453-1474; Yamamoto Y., Aiba H., baba T., Hayashi K., Inada T., Isono K., Itoh T., Kiura S., Kitagawa M., Makino K. (1997) Construction of a contigous 874-kb sequence of the Escherichia coli K-12 genome corresponding to 50.0-68.8 min on the linkage map and analysis of its sequence features. DNA Res., 4:91-113], ascription of function to protein product of all genes has not been so far achieved [Mori H., (2004) From the sequence to Cell modelling: Comprehensive Functional Genomics In Escherichia coli. Journal of Biochemistry and Molecular Biology, 37:83-92]. First reason is, that disabling of function of particular gene may not result in the expected breaking of given metabolic pathway. Second is the fact that functions of genes are not fully known may cause intervention to result in unexpected and undesirable disturbance in interaction of metabolic pathways not directly connected with the gene which is being disabled.

Third reason is the fact, that the mutation is being introduced into *E. coli* strain BL21(DE3), which is significantly modified in comparison to wild *E. coli* strain. It bears a number of mutations in its chromosome. These are mutations in *Ion* and *ompT* genes. Additionally, as mentioned above, this strain has introduced into its chromosome a gene encoding T7 phage RNA polymerase controlled by *lacUV5* promoter recognised by bacterial RNA polymerase. Disabling another gene, especially a gene from the group of genes, whose products are necessary for survival of the cell, can lead to notable disturbance in the cells life processes. Consequence of the performed actions may be obtaining of a strain unsuitable for the intended use, which is obtaining efficient expression of recombinant protein.

Below are described attempts to delete one of the genes, from the group of genes whose product is necessary for survival of the cell, which resulted in a failure. This confirms the fact, that the choice of the gene, whose deletion is being performed in order to obtain a host for stable expression is neither obvious nor random.

An example of a gene whose deletion has been attempted in order to obtain an advantegous strain for developed expression system is asd gene encoding aspartic β-semialdehyde dehydrogenase. Aspartate β-semialdehyde dehydrogenase is an enzyme common for biosynthesis pathways of amino acids from the family of aspartic acid (lysine, threonine, methionine, asparagine). This protein is encoded by asd gene, which according to literature data is present in a single copy in the *E. coli* bacteria chromosome. The conducted experiments clearly demonstrated, that inability to obtain asd mutants *of E. coli* BL21 (DE3) strain was a result of erroneous experiment planning To this end there was in parallel performed asd mutagenesis of *E coli* strains MG1665, TOP10F' and BL21(DE3). *E. coli* mutants in asd gene have been obtained and cultivated each time for *E coli* strains MG1665, TOP10F', but never for BL21(DE3). One of consequences of this mutation is overproduction of mucopolysaccharides by bacteria cells [Philips i wsp. 1984]. Subjected to mutagenesis asd gene encodes a protein involved in diaminopimelic acid biosynthesis pathway. This compound is present in the bacteria cell wall, similarly as mucopolysaccharides. It is possible that introduction of mutation in another gene of *E. coli* strain BL21(DE3) connected to construction of the cell wall of these bacteria would be to great burden for the cell.

Proper selection factor necessary for survival of the host cell is a protein factor, whose lack of expression in the cell causes cell's death. Such a defect can be a metabolic defect, which causes that one or more metabolites important for the cell's survival is not being synthesised.

Advantageous is a sequence encoding a protein selection factor chosen from a group containing the factor, especially protein, protein factor or hybrid proteins, which complement host's genetic defect, or their functional fragment.

By functional fragment is meant part of the gene encoding polypeptide or protein possessing the qualities of the selection factor.

Upstream of the 5' end of the sequence encoding the selection factor, whose role is performed by EPSPS, product of aroA gene, there has been placed non-coding region of this gene containing Sine-Dalgarno (SD) sequence, or its synthetic equivalent. Initially this sequence for aroA gene has been defined basing on literature data [Dunkan K., Coggins J. R. The serC-aroA operon of Escherichia coli A mixed function operon encoding enzyme from two different amino acid biosynthetic pathways. (1986) Biochem. J.]. It turned out, that it is not possible to obtain optimal level of EPSPS protein using this sequence. The region containing SD sequence before aroA gene has been established experimentally, additionally introducing modifications of 5' end of this region. These procedures allowed obtaining expression levels of the selection level optimal from the point of view of functioning of the developed system, i.e. selection factor expression level high enough to allow survival of the host cell, and at the same time sufficiently low for its simultaneous expression with the target protein not to cause lowering of the expression of the target protein.

In order to establish convenient non-coding sequence before aroA gene comprising SD sequence, functioning of the system has been tested with 4 different sequences added to 5' of sequence encoding selection factor: SEQ. ID NO 17, 18, 19, 20.

The use of sequences SEQ. ID NO 18, 19 turned out to be advantageous from the point of view of the invention.

Examples of sequences encoding selection factor complementing genetic defect of the host are sequences encoding e.g. proteins necessary for the host cell to survive. These can be proteins complementing auxotrophy of the bacteria.

Advantageous is the sequence of aroA gene encoding EPSPS.

Especially advantageous is the aroA sequence encoding EPSPS resistance factor containing non-coding region upstream of aroA gene including Shine-Dalgaro sequence modified in such a way, that it is recognised on mRNA by ribosomes resulting in optimal EPSPS expression level.

Suitable transcription terminator sequence from promoters other than phage promoters is a terminator able to prevent expression of the selection marker, which is required for survival of the host cell, by promoters present at the expression vector and recognised by polymerases of the host bacteria.

Sequence of transcription terminator can be natural or synthetic. It is preferred if upstream of 5'-end of transcription terminator there is a translation stop codon.

In a preferred example expression cassette includes tryptophan terminator [Molecular Cell Biology Darnell J. Loodish H., Baltimore D.] whose sequence is shown in bold italics in fig. 7. The term tryptophan terminator means transcription termination sequence from tryptophan operon (*tryp t).*

The cassette of the invention may contain promoters of other phages, especially similar to T7. Preferred is a promoter selected from a group comprising T7, T3, T5 and SP6 phage promoters, especially T7. The cassette according to the invention can contain promoters phages not belonging to T7 phage family, e.g. promoter p_{L}.

Phages T3, T5 and SP6 are similar to T7 [Nucleic Acid Research, 2005, 33,19. Information theory based T7-like promoter models: classification of bacteriophages and differential evolution of promoters and their polymerases. Z. Chen, T. D. Schneider.]. They are lytic phages, encoding their own RNA polymerase, which is highly processive and recognises conservative sequences in the region between positions -17 and +6 nucleotides upstream of mRNA start point. Promoters of these phages are strong promoters for microorganisms and are basis of widely used systems for recombinant protein production.

The target polypeptide can be encoded by any sequence encoding polypeptide homologous or heterologous for host cell. It can be for example a protein possessing therapeutic, diagnostic or biocatalytic properties, also hybrid protein or a fragment of any of such proteins, released from the cell or remaining in the cell. The encoding sequence can be a structural gene, cDNA, a synthetic sequence or a semi synthetic sequence.

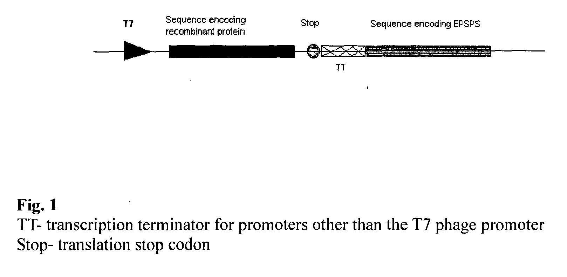

Construction of expression cassette as described in the invention is schematically presented in fig. 1. P denotes sequence of a phage promoter, X denotes sequence encoding the target polypeptide, S denotes translation stop codon, b is equal 0 or 1, T denotes transcription termination sequence for promoters other than phage promoters, M denotes sequence encoding selection marker which is required for survival of the host expressing the target protein.

In the expression cassette according to the invention nucleotide segment X-(S)_{b}-T-M is linked to phage promoter P.

Translation stop codon and transcription termination sequence of the host are located upstream of the 5' end of the sequence encoding the selection marker. The expression cassette ends with a transcription terminator characteristic of the phage, from which origins the promoter used in the expression vector.

Example of a favourable expression cassette is a unit which has a nucleotide segment whose sequence is shown as SEQ ID NO: 15, and also a cassette which has nucleotide segment whose sequence is shown as SEQ ID NO: 16.

The invention pertains also to the uses of the expression cassette which has phage promoter functionally linked to the sequence encoding target polypeptide and the sequence encoding selection marker, which is required for survival of the host expressing the target protein, where between the sequence encoding the target protein and the sequence encoding the selection marker, which is required for survival of the host, there is a transcription terminator from promoters other than phage promoters, in a construction of a vector for an expression system phage promoter/polymerase in a cell of a prokaryotic host.
It is favourable if at the 5' end upstream of transcription terminator from promoters other than phage promoters there is a translation stop codon.
The next subject of the present invention is an expression vector, characterised in that it contains an expression cassette consisting of a phage promoter functionally linked to a sequence encoding a target polypeptide as well as to a sequence encoding a selection marker, which is required for the survival of the host expressing the target polypeptide, where there is a transcriptional termination sequence for a promoter other than a phage promoter between the sequences encoding a target polypeptide and a selection marker, which is required for the survival of the host expressing target polypeptide.
Preferentially, there is a translation stop codon at the 5' end upstream of the transcriptional terminator for a promoter other than a phage promoter.

Methods of ligation of expression cassette elements and introduction of them into a vector proper for cloning and expression in a prokaryotic host and able to replicate in those organisms, are known.
There are also known methods of introduction of properly designed DNA fragments into bacterial cell in such a way, that *via* homologous recombination a deletion in chromosome is obtained.

To construct a vector as described in the invention it is convenient to use a plasmid vector. Such vectors are known to professionals and usually contain at least one of the following elements: a sequence or several sequences recognised by restriction endonucleases, a sequence enabling replication, a marker gene that enables selection of cells that contain the gene. A construction of expression cassette and a vector can process in following manner: 1) firstly, prepare sequence of the whole expression cassette and insert it into the vector or 2) elements of the expression cassette insert one by one into the proper vector (which may already contain some elements, for example a phage promoter sequence, transcription termination sequence or other).

Preferred is a plasmid vector containing a phage promoter, such as promoter of phage T7, T3, T5 or SP6, p_{L} especially T7, connected functionally with a sequence encoding the target polypeptide and a sequence encoding selection marker, such as a protein selection marker selected from a group containing marker complementing a genetic defect of the host, or their functional fragment. The sequence encoding the selection marker is preceded by a transcription terminator from promoters other than phage promoters and, preferably, a translation stop codon. An example is a vector with an expression cassette containing a nucleotide sequence shown as SEQ ID NO: 15 or with a cassette containing a nucleotide sequence shown as SEQ ID NO: 16.

According to variant of the invention is a sequence encoding selection marker, which is required for survival of the host expressing the target polypeptide, at the same time is the marker gene which enables selection of cells harbouring the vector.

Further subject of the invention is a prokaryotic host cell containing expression vector with a expression cassette containing phage promoter functionally linked to a sequence encoding target polypeptide and a sequence encoding selection marker, which is required to survival of the host expressing the target polypeptide, where between the sequence encoding the target polypeptide and the sequence encoding the selection marker, which is required to survival of the host, there is a transcription terminator from promoters other than phage promoters.

It is preferable, if upstream of the transcription terminator from promoters other than phage promoters, there is a translation stop codon.

A suitable host is a prokaryotic cell harbouring a gene encoding an RNA polymerase recognising a phage promoter.

This can be an *E. coli* cell. It can also be an *E. coli* cell with a defect in the ASD gene encoding aspartate-β-semialdehyde dehydrogenase.

The gene encoding the RNA polymerase recognising the phage promoter can be included in the prokaryotic host's genome. An example is an *E. coli* strain BL21 (DE3).

The gene encoding the RNA polymerase recognising the phage promoter can be incorporated into a phage or plasmid vector. This vector is then introduced into a prokaryotic host, without incorporating it into the genome of the host. This vector can be also a vector as described in the invention.

The vector as described in the invention is introduced into the prokaryotic host cell using known means.

The invention also relates to a method for production of the polypeptide, which consists in cultivating prokaryotic host cells comprising a) an expression vector with an expression cassette containing a phage promoter functionally linked to a sequence encoding the target polypeptide, which is required for survival of the host expressing the target polypeptide, where between the sequence encoding the target polypeptide and the sequence encoding the selection marker, which is required for survival of the host cell, there is a transcription terminator from promoters other than phage promoters, and b) a gene encoding an RNA polymerase recognising the phage promoter. The culturing is conducted in conditions which enable expression of the target polypeptide and the selection marker, and at the same time inhibit growth and cause elimination of cells in which there is no expression controlled by the phage promoter.

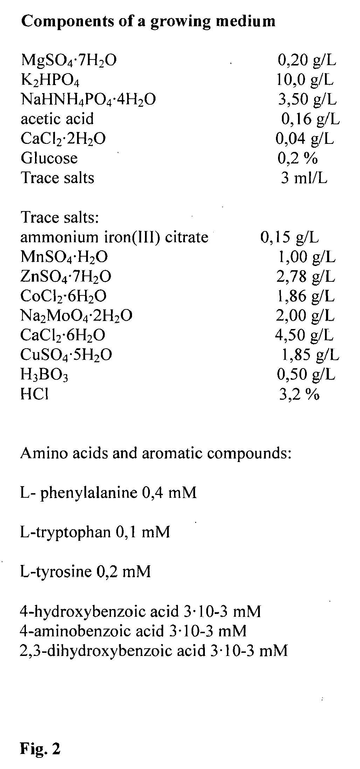

According to the invention a factor causing elimination of cells, which lack expression controlled by the phage promoter, is medium lacking specific component necessary for survival of these cells. It is optimal medium, there also can be used medium containing components necessary for survival, so called full medium. In case of culturing in full medium with the use of the described system, there has also been observed selection of cells stably expressing the target protein.
If the selection marker is a protein factor complementing a genetic defect of the host cell, then the factor causing death of the host cell or significant slow down of its division, if the selection marker is not expressed, will be the medium lacking the factor complementing the genetic defect of the host cell it is a medium not containing aromatic amino acids: L-tryptophane, L- tyrosine i L-phenyloalanine and aromatic compounds: 4-hydroxybenzoic acid, 4-aminobenzoic acid, 2,3-dihydroxybenzoic acid, alternatively a full medium, In the method according to the invention there are used conventional media for cell culturing chosen according to the used host organism. For *Escherichia coli* it can be rich medium such as one, whose composition has been originally researched during experiments conducted in order to develop the described invention. Composition of the medium is presented in Fig 2. Advantage of cultivation using this media, especially large-scale cultivation, is its relatively low cost, and its ability to ensure satisfactory levels of growth (comparable to one obtained for media used for laboratory-scale culturing).

Unexpectedly, in the course of the conducted experiments it was demonstrated, that the described system for stable expression without the use of antibiotics performs its function in full media. Target protein expression level has been researched in a culture employing the described invention. This culture has been using VB-MM medium with addition of aromatic amino acids: L-tryptopane, L-tyrosine i L-phenyloalanine and the aromatic compounds: 4-hydroxybenzoic acid, 4-aminobenzoic acid, 2,3-dihydroxybenzoic acid. Also in this case was observed selection of cells stably expressing the target protein. It can be assumed, that the observed selection during culturing in such media results from the fact, that *E.coli* BL21(DE3) cells as a result of the introduced deletion of the gene encoding EPSPS are not able to produce several other important metabolites apart of the named above aromatic compounds and aromatic amino acids. The full media would then supply the aromatic amino acids and aromatic compounds necessary for survival. The other missing metabolites can be produced by the cell using pathways other than the pathway using EPSPS. The results of culturing in media supplemented with above mentioned aromatic compounds and aromatic amino acids demonstrate, that for *E. coli* it is advantageous to overexpress the target protein when mutually EPSPS is produced and therefore maintaining operation of DPSPS-dependent metabolic pathways, rather than to produce the missing metabolites using alternative pathways.

It is preferred if the gene encoding the phage RNA polymerase is controlled by an inducible promoter, and therefore the processes of polymerase production and expression of the target polypeptide can be controlled. In this case expression is induced by addition of an inductor, such as IPTG (Isopropyl β-D-1-thiogalactopyranoside) or other known inductor.

In an expression system employing a phage promoter, especially T7, the polymerase is produced at a low level also without induction with IPTG. This phenomenon is described in the literature as "leakage". In the invention uses the phenomenon of "leakage", therefore there is eliminated the need for change of medium during the culturing of cells at the moment of induction. This ensures selection of cells from the very beginning of the culturing. Therefore, selection in this system plays two roles: it allows selection of host cells bearing the expression vector (selection marker function) and these, which produce the target polypeptide (selection factor function), necessary for survival of the host cell. Employment of the "leakage" phenomenon there is no need for additional procedures of enriching culture media at the initial culture stages before the induction.

The invention lets to obtain several times higher amounts of the target polypeptide from the given volume of culture and to maintain a high and stable in time levels of production, which also lowers cost of production, including the cost of protein purification. Elimination from the culture cells not producing the target polypeptide, which would grow and divide faster, prevents the culture to be dominated by these cells.

The described invention is a more advantageous solution of the stable expression problem than the invention described in patent application PL379905 (published 2007-12-10). Its advantage lies in its ability for much longer culturing of bacteria while simultaneously maintaining stable expression of the target protein. Therefore, significantly larger amount of the final product is being obtained from the given volume of media. In the invention described in the patent application PL379905 (published 2007-12-10) the selection factor ensuring stable expression is kanamycin resistance. Imperfection of that stable expression system of the target protein, apart of the necessity to use antibiotics, are naturally appearing spontaneous mutants resistant to kanamycin. Such mutants do not produce the target protein and dynamically "overgrow" the culture.

In the described solution of the problem the possibility of appeareance of spontaneous mutants has been eliminated. In the developed system the culture after immobilisation of the cells expressing simultaneously the target protein and the protein necessary for survival of the cell, can be conduced as batch culture, semi-continuous culture, continuous culture.

The invention has been described using figures and sequence lists, where:
Fig. 1 represents an expression cassette, said nucleotide sequence "Kes" is cloned into NdeI and HindIII restriction sites of pIG expression vector.
Fig. 2 represents composition of growing media.
Fig. 3 present electrophoretical separation of lysates of bacteria samples taken in experiment 1.

### Lines:

LMW-Molecular weight protein markers for electrophoresis. It contains 97, 66, 45, 30, 20.1 and 14.4 kDa polypeptides.

1, 4, 7, 10 - Cell lysate of bacteria *E. coli* B121(DE3) strain transformed with pIGKesAroAPA4D-G vector. Bacteria were cultured in VB-MM. Samples of culture were taken every 2, 8, 10, and 21 hours fallowing the induction with IPTG.

2, 5, 8, 11 - Cell lysate of bacteria *E. coli* Bl21(DE3)ΔaroA strain transformed with pIGKesAroAPA4D-G vector. Bacteria were cultured in VB-MM. Samples of culture were taken every 2, 8, 10, and 21 hours fallowing the induction with IPTG.

3, 6, 9, 12 - Cell lysate of bacteria *E. coli* B121(DE3)ΔaroA strain transformed with pIGKesAroAPA4D-G vector. Bacteria were cultured in VB-MM+aa. Samples of culture were taken every 2, 8, 10, and 21 hours fallowing the induction with IPTG.

In order to better explanation of the invention the example are presented below..

### Examples

### Example I. Construction of strain for expression, expression cassette and expression vectors.

### Construction of E. coli BL21(DE3)ΔaroA strain.

*E. coli* BL21(DE3) strain was used for the construction of *E. coli* BL21(DE3)ΔaroA strain.The functional deletion of the *E. coli* BL21(DE3) *aroA* gene encoding 3-phosphoshikimate 1-carboxyvinyltransferase (EPSPS) was performed. In order to obtain *E*. *coli* BL21(DE3) strain with the mutation in aroA gene linear DNA fragments comprising neomycin phosphotransferase II (NPTII) gene were used. The gene is flanked with homology regions to the external parts of *aroA* gene. Obtained linear DNA fragments were introduced into BL21(DE3) strain *E. coli* by electroporation.

The critical/crucial step of this procedure is to use highly competent cells of *E. coli* BL21(DE3) strain. Bacteria cells were transformed with the fragments using Gene-Pulser X-Cell electroporator Bio-Rad, 2 mm electroporation cuvettes, the pulse parameters: 2.5 kV, 25 μF, 200 Ohms. After electroporation cells were plated on medium containing aromatic amino acids: L-phenylalanine, L-tryptophan, L-tyrosine, and aromatic compounds: 4-hydroxybensoic acid, 4-aminobensoic acid, dihydroxybensoic acid (show on the Fig. 2) and kanamycin. Bacterial colonies were tested in PCR reaction for gene encoding NPTII inserted into the chromosome and_flanked by sequence of *homology* to the target *site.* Primers ZaAROA, PrzeAROA and Aro700R SEQ. ID NO: 5, 6, 7. Obtaining of bacteria with NPTII gene introduced into the chromosome was confirmed by the electrophoretical separation of the PCR mixture in agarose gel and sequencing of amplified and isolated DNA fragments.

The NPTII gene was introduced into the specific site of the chromosome, flanked by sequence of homology to the target site. The results confirmed that *E. coli* BL21(DE3) with the deletion of *aroA* gene was successfully obtained.

### Construction of expression cassette

The *E. coli* BL21(DE3) strain was used in PCR (PCR1) to amplify, with the use of AroA1 (sense primer, sequence shown in SEQ. ID NO: 1) and AroAk (reverse primer sequence shown in SEQ. ID NO: 3) complete nucleotide sequence of aroA gene (1284 bp). A fragment of primers AroA1 and AroAk were designed on the basis of aroA gene sequence. The sense primer AroA1 introduces at the 5'end of DNA molecule sequence homological to expression cassette sequence, the antisense primer AroAk introduce at the 3'end of DNA molecule recognition site for HindIII restriction nuclease and alternation of stop codon (TGA to TAA). Following the PCR the mixture was separated electrophoretically on an agarose gel. The amplified DNA fragment was eluted from the agarose gel, and used as a template in the next PCR reaction (PCR2).

The product of PCR1 was used as a template in PCR2 to amplify with the use of AroA2 (sense primer, sequence shown in SEQ. ID NO: 2) and AroNdeD (reverse primer sequence shown in SEQ. ID NO: 4) 206 bp fragment. The sense primer AroA2 introduces at the 5'end of DNA molecule sequence homological to expression cassette sequence and recognition site for SalI restriction nuclease. The antisense primer AroNdeD introduce nucleotide alternation of thymine to cytosine at the position 156. This modification ma aims to remove from the sequence a sequence recognised by restriction nuclease Ndel. The modification does not change the amino acid sequence. Following the PCR2 the mixture was separated electrophoretically on an agarose gel. The amplified DNA fragment was eluted from the agarose gel, and used as a template in the next PCR reaction (PCR3).

The fragment from PCR1 was used as a template in PCR3 to amplify with the use of AroAk and the product of PCR2 as primers 1369 bp fragment. Following the PCR3 the mixture was separated electrophoretically on an agarose gel. The amplified DNA fragment was eluted from the agarose gel and digested with the restriction nuclease SalI and HindIII and deproteinised. The obtained nucleotide unit constitutes an expression cassette "KesAroA" comprising translational stop codon, transcriptional termination sequence from tryptophan operon and aroA gene. The resultant fragment was ligated with NdeI and HindII-digested and deproteinized vector. The validity of the sequence introduced into the vector was confirmed by sequencing. As a result of the procedure the pIGKesAroA vector was obtained. It is the vector with KesAroA expression cassette introduced under transcriptional control of the T7. The cassette was cloned to a vector selected from a group comprising multiple cloning site, and promoter recognized by the RNA polymerase from T7 phage and transcriptional stop codon from T7 phage.

### Construction of pIGKesAroAPA vector

The plasmid comprising the complete nucleotide sequence of *Bacillus anthracis* protective antigen domain 4 was used in PCR reaction to amplified, with the use of PA-4DP (sense primer, sequence shown as SEQ ID NO: 11) and PA4DKXho primer (reverse primer sequence shown as SEQ ID NO: 13), 429 bp DNA fragment according to coordinates of GeneBank sequences of the gene (accession No. AF065404). The PA-4DP and PA4DKXho primers were designed on the basis of coding sequence of *Bacillus anthracis* protective antigen domain 4. The sense primer introduces recognition sites for EcoRI and Ndel restriction nucleases, the antisense primer introduce recognition site for SalI restriction nuclease. Following the PCR the mixture was separated electrophoretically on a polyacrylamide gel. The amplified DNA fragment was eluted from the polyacrylamide gel, and digested with the restriction nuclease NdeI and SalI and deproteinised. The obtained fragment was ligated with the vector pIGKesAroA, digested with the same restriction nucleases and deproteinised. The validity of the sequence introduced into the vector was confirmed by sequencing. As a result of the procedure the pIGKesAroAPA vector with the expression cassette comprising nucleotide PA-4D+Stop+Term+aroA unit under transcriptional control of the T7 promoter was obtained.

### Construction of pIGKesAroAPA-G vector

The plasmid pIGKesAro was used in PCR (PCR1) to amplify with the use of AroA1zG (sense primer, sequence shown as SEQ ID NO: 10) and AroAk primer (reverse primer sequence shown as SEQ ID NO: 3), fragment comprising the complete nucleotide sequence of aroA gene (1284 bp). The AroA1zG and AroAk primers were designed on the basis of coding sequence of aroA gene. The sense primer AroA1zG introduces at the 5'end of DNA molecule sequence homological to expression cassette sequence, the antisense primer AroAk introduce at the 3'end of DNA molecule recognition site for HindIII restriction nuclease and alternation of stop codon (TGA to TAA). Following the PCR the mixture was separated electrophoretically on an agarose gel. The amplified 1326 bp-long DNA fragment was eluted from the agarose gel, and used as a template in the next PCR reaction (PCR2).

The product of PCR1 was used as a template in PCR2 to amplify with the use of AroA2 (sense primer, sequence shown in SEQ. ID NO: 2) and AroAk a DNA fragment. The sense primer AroA2 introduces at the 5'end of DNA molecule sequence homological to expression cassette sequence and recognition site for SalI restriction nuclease. Following the PCR2 the mixture was separated electrophoretically on an agarose gel. The amplified 1329 bp-long DNA fragment was eluted from the agarose gel, and used as a template in the next PCR reaction (PCR3).

The plasmid comprising the complete nucleotide sequence of *Bacillus anthracis* protective antigen domain 4 was used in PCR3 reaction to amplified, with the use of PA-4DP (sense primer, sequence shown as SEQ ID NO: 11) and PAzAroA primer (reverse primer sequence shown as SEQ ID NO: 12), a DNA fragment. The PA-4DP and PAzAroA primers were designed on the basis of coding sequence of *Bacillus anthracis* protective antigen domain 4. The sense primer introduces recognition sites for EcoRI and NdeI restriction nucleases, the antisense primer introduce recognition site for SalI restriction nuclease. the antisense primer introduce at the 3'end of DNA molecule sequence homological to 5'end of expression cassette sequence amplified in PCR2. Following the PCR3 the mixture was separated electrophoretically on a polyacrylamide gel. The amplified DNA fragment was eluted from the polyacrylamide gel. The fragment from PCR2 was used as a template in PCR4 to amplify with the use of AroAk and the product of PCR2 as primers 1800 bp fragment. Following the PCR4 the mixture was separated electrophoretically on an agarose gel. The amplified 1800 bp-long DNA fragment was eluted from the agarose gel and digested with the restriction nuclease NdeI and HindIII and deproteinised. Obtained nucleotide unit is formed by gene encoding protective antigen domain 4 and expression cassette "KesAroA" comprising nucleotide PA-4D+Stop+Term+aroA unit under transcriptional control of the T7 promoter. The obtained fragment was ligated with the vector digested with the same restriction nucleases and deproteinised. The validity of the sequence introduced into the vector was confirmed by sequencing. As a result of the procedure the pIGKesAroAPA-G vector was obtained. It is the vector with the expression cassette KesAroA and gene encoding protective antigen domain 4 under transcriptional control of the T7 promoter.

### Construction of pIGKesAroAPA-AGG, pIGKesAroAPA-Km vector

The construction of vectors pIGKesAroAPA-AGG, pIGKesAroAPA-Km was obtained in an analogous way as/to construction of pIGKesAroAPA-G. To construct these vectors AroA1-AGGAGG SEQ. ID NO: 9 primer was used instead of AroA1zG, as described in PCR1 section "Construction of pIGKesAroAPA-G vector", primer AroA1-AGGAGG SEQ. ID NO: 9 to construct pIGKesAroAPA-AGG vector and primer AroA1-SDKm SEQ. ID NO: 8 to construct pIGKesAroAPA-Km vector.

### Construction of vectors pIGT7KesPA (comparative vector)

The plasmid comprising the whole nucleotide sequence of *Bacillus anthracis* protective antigen domain four was used in PCR reaction to amplified, with the use of sense PA-4DP primer (sequence shown as SEQ ID NO: 11) and antisense PA4DK primer (sequence shown as SEQ ID NO: 14), 429 bp DNA fragment (according to coordinates of GeneBank sequences of the gene, accession No. AF065404). The PA-4D and PA4DK primers were designed on the basis of coding sequence of *Bacillus anthracis* protective antigen domain four. The sense primer introduces recognition sites for EcoRI and NdeI restriction nucleases, the antisense primer introduce recognition site for HindIII restriction nuclease. Following the PCR the mixture was separated electrophoretically on a polyacrylamide gel. The amplified DNA fragment was eluted from the polyacrylamide gel, and digested with the restriction nuclease NdeI and HindIII and deproteinised. The obtained fragment (sequence shown as SEQ ID NO: 21) was ligated with the pIGT7 vector , digested with the same restriction nucleases and deproteinised. The validity of the PA-4D sequence introduced into the vector was confirmed by sequencing. As a result of the procedure the pIGT7PA vector was obtained.

### Example. Expression system and its operation

Operation of the system in the presence of expression cassette in vectors according to the invention, in which expression is based on T7 phage promoter, has been tested for PA-4D protein originated from bacteria *Bacillus anthracis.*

Plasmid comprising the verified insert were transformed into *E.coli* cells strain BL21(DE3) and BL21(DE3)ΔaroA. In the chromosome of *E. coli* BL21(DE3) strain and its derivatives there is a gene encoding RNA polymerase of T7 phage under the control of *lacUV5* promoter. The gene is recognised by the bacterial RNA polymerase. There is an operator sequence between the promoter and the gene encoding T7 RNA polymerase which binds represor protein in that manner blocking the initiation of transcription. Only when represor binds to IPTG, which leads to loss of protein affinity to operator site, that the transcription of phage polymerase gene may start. Phage polymerase transcribe a gene in an expression vector under the control of promoter recognised by T7 bacteriophage RNA polymerase.

*E. coli* cells were transformed using electroporation. After transformation bacteria cells were plated on VB-MM solid medium and incubated 15-24 hours at 37°C. After incubation colonies of bacteria caring the plasmid was observed. Two *E. coli* strains were obtained:
1. BL21(DE3) caring: pIGKesAroAPA-AGG, pIGKesAroAPA-G, pIGKesAroAPA and/or pIGKesAroAPA-Km
2. BL21(DE3)AaroA bacteria caring: pIGT7PA, pIGKesAroAPA, pIGKesAroAPA-Km, and/or pIGKesAroAPA-AGG, pIGKesAroAPA-G.

After the transformation *of E. coli* BL21(DE3)ΔaroA with pIGKesAroAPA, pIGKesAroAPA-Km plasmids no growth on solid VB-MM medium was observed.

Lack of the growth *of E. coli* BL21(DE3)ΔaroA caring pIGKesAroAPA, pIGKesAroAPA-Kmplasmids on VB-MM medium is an evidence of lack or too low expression of EPSPS protein encoded by the aroA gene on a plasmid. Hence, there is no complementation of the genetic defect of the host cell, which enable bacteria growth. Do dalszych eksperymentów E. *coli* strain BL21(DE3)ΔaroA caring pIGKesAroAPA-AGG, and pIGKesAroAPA-G plasmids and *E. coli* strain BL21(DE3) caring pIGT7PA were used.

### Design of experiments

Two experiments were conducted in which:
Experiment 1
   1. Bacteria *E. coli* strain BL21(DE3)ΔaroA carrying p1GKesAroAPA-G plasmid were cultivated in VB-MM medium.
   2. Bacteria *E. coli* strain BL21(DE3)ΔaroA carrying pIGKesAroAPA-G plasmid were cultivated in VB-MM+aa medium.
   3. Bacteria *E. coli* strain BL21(DE3) carrying pIGKesAroAPA-G plasmid were cultivated in VB-MM medium.
   4. Bacteria *E. coli* strain BL21(DE3) carrying pIGT7PA plasmid were cultivated in VB-MM medium.
Experiment 2
   1. Bacteria *E. coli* strain BL21(DE3)ΔaroA carrying pIGKesAroAPA-AGG plasmid were cultivated in VB-MM medium.
   2. Bacteria *E. coli* strain BL21(DE3)ΔaroA carrying pIGKesAroAPA-AGG plasmid were cultivated in VB-MM+aa medium.
   3. Bacteria *E. coli* strain BL21(DE3) carrying pIGKesAroAPA-AGG plasmid were cultivated in VB-MM medium.
   4. Bacteria *E. coli* strain BL21(DE3) carrying pIGT7PA plasmid were cultivated in VB-MM medium.

The efficiency of the system modified according to the invention was compared to the unmodified system using PA-4D as a sample target protein. To this end conventional (pIGT7PA) vector comprising the T7 bacteriophafage promoter and sequence encoding PA-4D as well as the modified (pIGKesAroAPA-AGG and pIGKesAroAPA-G) vectors comprising the T7 bacteriophafage promoter and sequence encoding PA-4D as a part of the expression cassette were used.
*E. coli* BL21(DE3)ΔaroA and BL21(DE3) were used as host strains.
In the experiment 1 an expression level of PA-4D protein in *E.coli* BL21(DE3)ΔaroA harbouring pIGKesAroAPA-G in relation to an expression level of PA-4D obtained in protein in *E.coli* BL21(DE3) caring the same plasmid was examined. Bacteria were cultured in VB-MM medium. The control of expression level of PA-4D protein obtained in *E.coli* BL21(DE3)ΔaroA caring pIGKesAroAPA-G was conducted. Bacteria were cultured in VB-MM+aa medium (reach media).
Similarly, in the experiment 2 an expression level of PA-4D protein in *E.coli* BL21(DE3)ΔaroA harbouring pIGKesAroAPA-AGG in relation to an expression level of PA-4D obtained in protein in *E.coli* BL21(DE3) caring the same plasmid was examined. Bacteria were cultured in VB-MM medium. The control of expression level of PA-4D protein obtained in *E.coli* BL21(DE3)ΔaroA caring pIGKesAroAPA-AGG was conducted. Bacteria were cultured in VB-MM+aa medium (reach media).
In both experiments were verified the expression level of PA-4D protein obtained in the modified and unmodified T7 phage polimerase/promote system. Bacteria were cultured in VB-MM medium.
In both experiments there was verified the expression level of PA-4D protein obtained with the use of non-modified T7 phage polymerase/promoter expression system. This culture used VB-MM media.

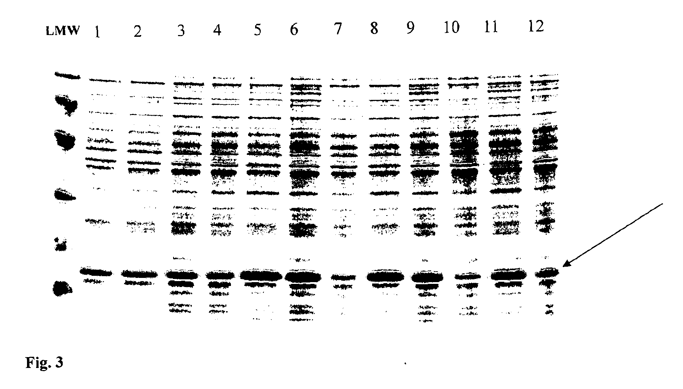

In each experiment the protein expression was conducted. To this end *E. coli* bacteria strain BL21(DE3) harbouring the recombinant plasmid were grown in 3 ml of VB-MM broth at 37°C for 3 hours, diluted with fresh VB-MM broth (1:100) and shaked at 37°C till A₆₀₀ reached 0.4. Next the target polypeptide was induced by addition of isopropylthiogalactoside (IPTG, 0.1 mM final concentration) and shaking was continued for 18 more hours. Samples for A₆₀₀ measurements and for a electrophoretical separation on polyacrylamide gel (SDS-PAGE) were taken every 2 hours in the time course of culturing conducted fallowing the induction. The presence and amount of target polypeptide was analysed by bacterial lysate separation on 15% polyacrylamide gel (SDS-PAGE) carried out as described by Laemmli [Laemmli UK. Cleavage of structurals proteins during the assembly of the head of bacteriophage T4. Nature. 1970 Aug 15;227(5259):680-5].
The separated proteins were visualised by staining with Coomassie Brillant Blue G. Electrophoretical separation of lysates of bacterial cell samples taken during the experiment 1 are shown in figure 3.
The level of expression of PA-4D protein has been marked with an arrow on the electrophoretic separation (Fig 3.). There is visible the stable expression level of this protein during the cultivation conducted with the use of the system according to the invention, when compared to expression level of this protein in the standard expression system using T7 phage polymerase/promoter.
The difference in the expression level is already seen in lines of electrophoretically separated lysates of bacteria taken 8 hours after induction of target protein expression. This difference in expression levels grows as time passes. The difference in expression levels steadily grows and advantage of *E.coli* BL21(DE3)ΔaroA caring the pIGKesAroAPA-G over *E.coli* BL21(DE3) caring the pIGKesAroAPA-G plasmid increases in time.

Additionally, in this experiment the expression level of target polypeptide obtained in commonly used system containing solely promoter of T7 phage was compared with the expression level of the target polypeptide obtained in the system according the invention. To this end two vectors were used: the standard vector with T7 phage promoter and with cloned sequence encoding PA-4D (pIGT7PA) and the modified vector (pIGKesAroAPA-AGG and pIGKesAroAPA-G) comprising PA-4D coding sequence as a part of nucleotide unit of the cassette according to the invention. Analysis of electrophoretical separations of samples taken at early stages of cultivation (first 4 hours after induction of expression of the target polypeptide) shows that expression of the target protein is on the same level in the standard system employing T7 phage promoter and in the system described in the invention. It is possible because in the system according to the invention high level of expression, typical for T7 phage promoter, is directed mainly at the target promoter.

In the process conducted as described in the invention bacteria cells in whose chromosome occurred a mutation causing lack of production of functional T7 phage polymerase and/or bacteria in which, due to a mutation, T7 phage promoter lost its functionality, are eliminated from the culture. Functional polymerase and a T7 phage promoter recognised by that polymerase are prerequisites for obtaining expression of the target polypeptide. This means, that from the culture are eliminated bacteria cells not producing the target polypeptide, which otherwise would grow and divide faster. Therefore the invention allows to prevent the culture from being dominated by the bacteria not expressing the target polypeptide.

## Claims

1. A prokaryotic host cell containing an expression cassette containing a phage promoter and a sequence encoding the target polypeptide, where the cassette has the formula:
P-X-S-T-M
where: P denotes the T7 phage promoter sequence, X denotes the target polypeptide sequence, S denotes the translation stop codon, T denotes transcription terminator for promoters other than the T7 phage promoters and M denotes the sequence encoding the selection factor necessary for survival of the host expressing the target polypeptide and complementing genetic defect of the host cell, the sequence encoding the selection factor is the aroA sequence encoding 5-enolpyruvylshikimate-3-phosphate synthase (EPSPS), and the host for the vector containing the cassette is E. coli strain BL21 (DE3) with functional deletion of the aroA gene.

2. The host cell according to claim 1, **characterised in that**, the transcription terminator for promoters other than the phage promoter is a tryptophan terminator.

## Patentansprüche

1. Prokaryontische Wirtszelle, enthaltend eine Expressionskassette enthaltend einen Phagenpromotor und eine Sequenz, die für das Zielpolypeptld kodiert, wobei die Kassette die Formel:
P-X-S-T-M
aufweist, worin: P die T7 Phagenpromotorsequenz bezeichnet, X die Zielpolypeptidsequenz bezeichnet, S das Translations-Stoppkodon bezeichnet, T den Transkriptionsterminator für Promotoren anders als den T7 Phagenpromotor bezeichnet, und M die Sequenz bezeichnet, die für den Selektionsfaktor kodiert, der für das Überleben des Wirtes der das Zielpolypeptid exprimiert und einen genetischen Defekt der Wirtszelle komplementiert erforderlich ist, wobei die Sequenz, die für den Selektionsfaktor kodiert, die aroA Sequenz ist, die für 5-Enolpyruvylshikimat-3-phosphat-Synthase (EPSPS) kodiert, und der Wirt für den Vektor der die Kassette enthält E. coli Stamm BL21 (DE3) mit einer funktionellen Deletion des aroA Gens ist.

2. Wirtszelle nach Anspruch 1, **dadurch gekennzeichnet, dass** der Transkriptionsterminator für Promotoren anders als den Phagenpromotor ein Tryptophan-Terminator ist.

## Revendications

1. Cellule hôte procaryote contenant une cassette d'expression contenant un promoteur de phage et une séquence codant pour le polypeptide cible, où la cassette répond à la formule :
P-X-S-T-M
où : P représente la séquence promotrice de phage T7, X représente la séquence polypeptidique cible, S représente le codon d'arrêt de traduction, T représente le terminateur de transcription pour les promoteurs autres que les promoteurs de phage T7 et M représente la séquence codant pour le facteur de sélection nécessaire à la survie de l'hôte exprimant le polypeptide cible et complétant le défaut génétique de la cellule hôte, la séquence codant pour le facteur de sélection est la séquence aroA codant pour la 5-énolpyruvylshikimate-3-phosphate synthase (EPSPS), et l'hôte pour le vecteur contenant la cassette est la souche d'E. coli BL21 (DE3) avec une délétion fonctionnelle du gène aroA.

2. Cellule hôte selon la revendication 1, **caractérisée en ce que** le terminateur de transcription pour les promoteurs autres que le promoteur de phage est un terminateur de tryptophane.
